# EUROPEAN PATENT APPLICATION

(11) **EP 2 561 846 A1**
(43) Date of publication of application: **27.02.2013**
(21) Application number: 11772016.9
(22) Date of filing: 19.04.2011
(51) Int. Cl.: A61F 13/496

(54) **ABSORBENT ARTICLE**

(30) Priority: 19.04.2010 JP 2010096532
(71) Applicant: Unicharm Corporation, Ehime 799-0111 (JP)
(72) Inventor: MUKAI, Hirotomo, Kanonji-shi, Kagawa 769-1602 (JP); WATABE, Yoshihisa, Kanonji-shi, Kagawa 769-1602 (JP)
(74) Representative: Peter, Julian
(86) International application number: PCT/JP2011/059650
(87) International publication number: WO 2011/132681

(57) **Abstract**

In the disclosed absorbent article (1), in a front torso-surrounding region (S10A), the distance (D1) between the elastic member (100A) that is most towards leg-surrounding openings among a plurality of elastic members that extend roughly linearly in the widthwise direction (W) and the edges (E1) that are on the leg-surrounding opening (P) side of the front torso-surrounding region (S10A) is configured in a manner so as to gradually shorten and then gradually lengthen when going from both side edges (C) towards the center in the widthwise direction (W).

## Description

### TECHNICAL FIELD

The present invention relates to an absorbent article.

### BACKGROUND ART

Conventionally, there are known a chassis having a front waistline portion, a rear waistline portion, and a crotch portion, and an absorbent article having an absorbent body provided from the crotch portion across the front waistline portion and rear waistline portion.

A technique is known (for example, see Patent Literature 1) according to such an absorbent article, by arranging a plurality of elastic members for leg holes extending approximately linearly in the width direction of the absorbent article, in the front waistline portion, the length of the elastic members for the leg holes is shortened, thus reducing the cost.

### CITATION LIST

### PATENT LITERATURE

[Patent Literature 1] Japanese Patent Publication No. 2005-58511

### SUMMARY OF INVENTION

However, the applicants faced the following problem as regard the above absorbent article.

In the absorbent article having the aforementioned configuration, because both the elastic members for the leg holes and the edge at the leg hole opening side in the front waistline portion are almost linear, when the elastic members for the leg holes get deformed, the portion where the elastic members are not arranged and which lies between the edge at the leg hole opening side of the portion in which the elastic embers for the leg holes are arranged and the edge at the leg hole opening side of the front waistline portion cannot cover the neighboring part of the inguinal portion while fitting the body of the wearer, and therefore, the problem was that the wearer did not have a pleasant feeling of wearing the absorbent article.

Furthermore, the absorbent article having the aforementioned configuration has a problem in that, in order to reduce the cost, when the basis weight of the waistline sheet is reduced, the problem of breakage of the joined portion in the both-side edges, particularly, the exertion of force at the upper and lower ends of the joined portion resulting in the occurrence of breakage from the portion was seen.

Thus, the present invention has been achieved in view of the aforementioned problem, and an object thereof is to provide an absorbent article by which it is possible to eliminate the discomfort caused by a decline in the feeling of wearing the absorbent article while shortening the length of the elastic members for the leg holes, and to cover the neighboring part of the inguinal portion of the wearer.

A first feature of the present invention is summarized as an absorbent article, comprising: a chassis; and an absorbent body, wherein the chassis includes a front waistline portion, a rear waistline portion, and a crotch portion provided between the front waistline portion and the rear waistline portion, respective one of both-side edges of the front waistline portion and respective one of both-side edges of the rear waistline portion are configured to be joined, and in the front waistline portion, a distance between an elastic member closest to a leg hole opening from among a plurality of elastic members extending in an almost straight line in a width direction and an edge at the leg hole opening side of the front waistline portion is configured to increase gradually after decreasing gradually from the both-side edges towards a center in the width direction.

### BRIEF DESCRIPTION OF DRAWINGS

[Fig. 1]Fig. 1 is an external view of an absorbent article according to a first embodiment of the present invention.
[Fig. 2] Fig. 2 is an exploded view of the absorbent article according to the first embodiment of the present invention.
[Fig. 3]Fig. 3 is a plan view of the absorbent article according to the first embodiment of the present invention.
[Fig. 4]Fig. 4 is a cross-sectional view along a line A-A in the plan view of the absorbent article according to the first embodiment of the present invention.
[Fig. 5] Fig. 5 is a cross-sectional view along a line B-B in the plan view of the absorbent article according to the first embodiment of the present invention.
[Fig. 6] Fig. 6 is an external view in which a state when wearing the absorbent article according to the first embodiment of the present invention is seen from the lateral side.
[Fig. 7] Fig. 7 is a view for explaining a method of manufacturing the absorbent article according to the first embodiment of the present invention.
[Fig. 8]Fig. 8 is an exploded view of the absorbent article according to a first modification of the present invention.

### DESCRIPTION OF EMBODIMENTS

### (First embodiment of the present invention)

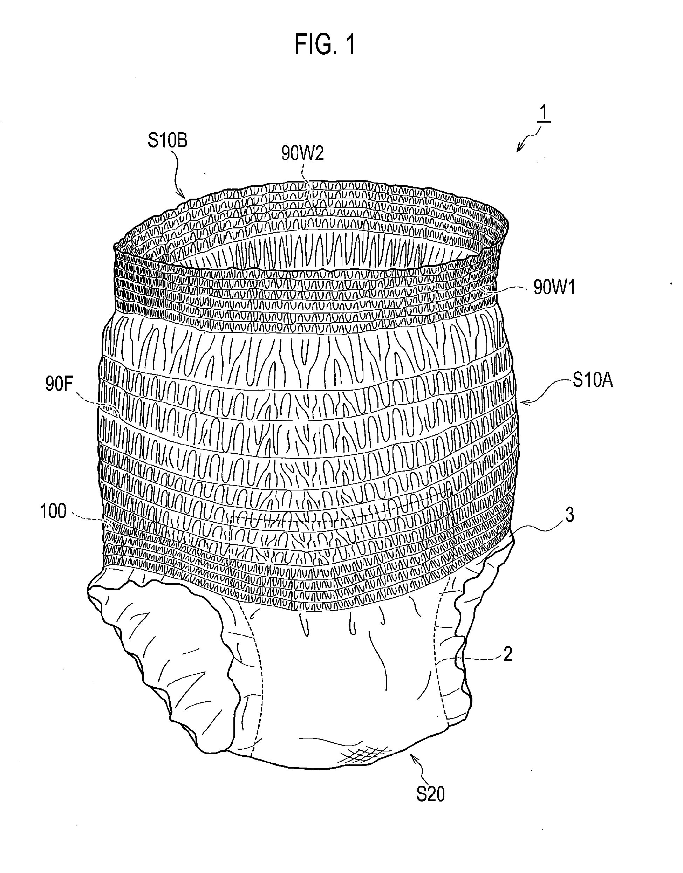

With reference to Fig. 1 to Fig. 7, an absorbent article 1 according to a first embodiment of the present invention will be described. The absorbent article 1 according to the embodiment is a pant-type diaper, a water-absorbent underwear, etc.

Fig. 1 is an external view of the absorbent article 1 according to the embodiment. As shown in Fig. 1, the absorbent article 1 according to the embodiment has a chassis 3 and an absorbent body 2.

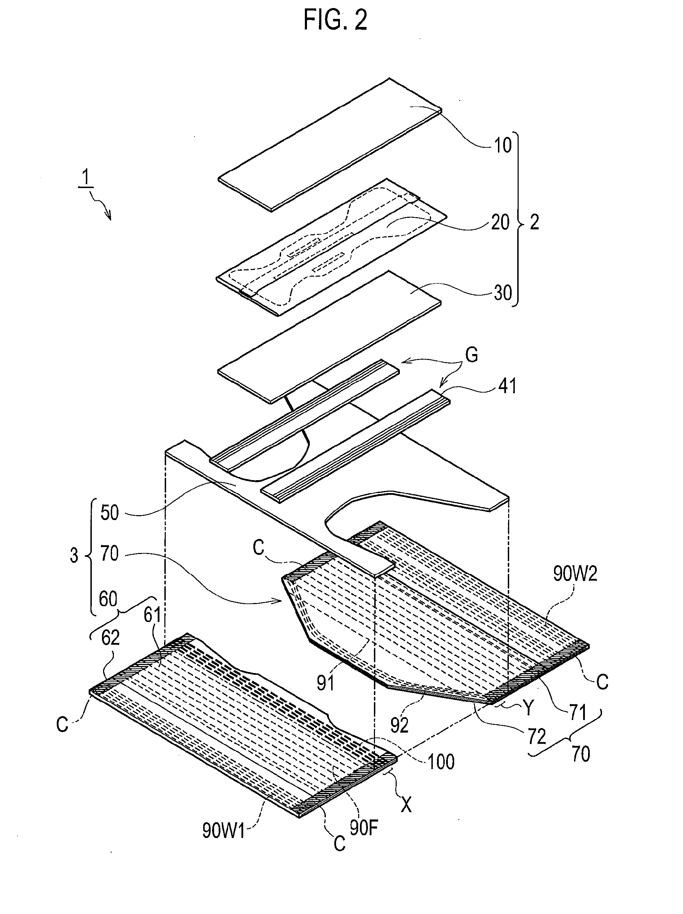

Specifically, as shown in Fig. 2 to Fig. 5, the absorbent body 2 is configured by an absorbent body side topsheet 10 that is a liquid-permeable sheet in contact with the skin of the wearer; an absorber core 20; and an absorbent body side backsheet 30 that is a liquid-impermeable sheet.

A sheet made from a hydrophilic nonwoven cloth made of fibers such as polyolefin and polyethylene terephthalate (PET), which is manufactured by methods such as spun bonding and air-through can be used as the absorbent body side topsheet 10. A sheet made of a water-resistive film such as polyethylene (PE) can be used as the absorbent body side backsheet 30.

For example, the absorbent body side topsheet 10 is 25 g/m² of air-through nonwoven cloth, and the absorbent body side backsheet 30 is 22 g/m² of moisture-permeable polyethylene film.

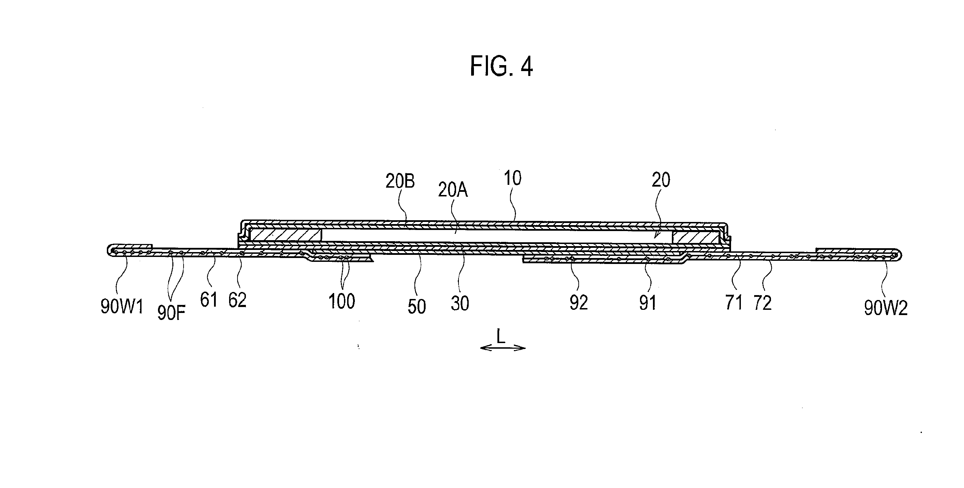

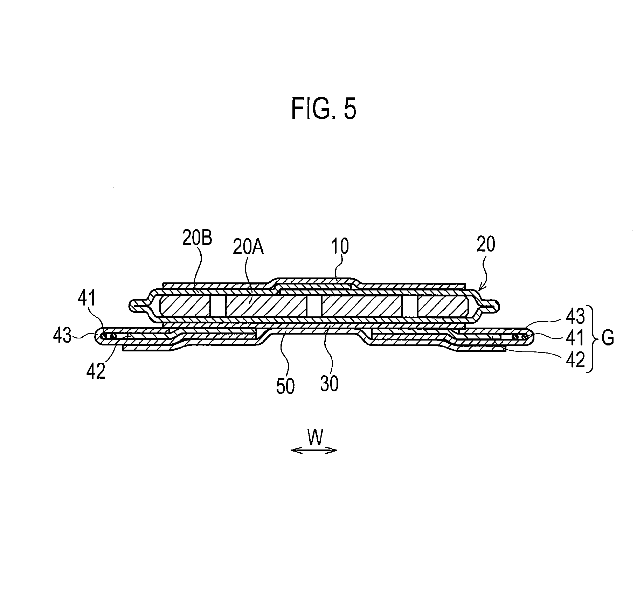

The absorber core 20 is generated, for example, by wrapping a mixture 20A of ground pulp (for example, 200 g/m²) and a high absorbent polymer (for example, 200 g/m²) with a wrapping sheet 20B, as shown in Fig. 4 and Fig. 5.

A sheet made from a hydrophilic nonwoven cloth made of fibers such as polyolefin and polyethylene terephthalate, which is manufactured by methods such as spun bonding and air-through can be used as the wrapping sheet 20B. For example, the wrapping sheet 20B is 13 g/m² of SMS nonwoven cloth.

The absorbent body side topsheet 10, the absorbent body side backsheet 30, and the wrapping sheet 20B are mutually joined by a hot-melt adhesive (for example, spiral HMA).

For example, the basis weight of the hot-melt adhesive is 1.5 to 10 g/m². Furthermore, besides spiral coating, methods such as slot coating, control seam, beading, and curtain coater may be used for joining by the hot-melt adhesive.

Note that the absorber core 20 is a thin absorber with a thickness of approximately 2.0 mm, and with less ruggedness on the surface thereof.

As shown in Fig. 2 to Fig. 5, a barrier cuffs G is provided at both sides in the longitudinal direction L of the absorbent body 2. In other words, the barrier cuffs G is joined with both sides in the width direction W of the absorbent body side backsheet 30.

The barrier cuffs G is configured by thread-shaped elastic members 41 for the barrier cuffs, a water-resistant film 42, and a hydrophobic nonwoven cloth 43.

As shown in Fig. 5, at the upright supporting point side (that is, the inner side in the width direction W) of the barrier cuffs 40, the position of the end of the water-resistant film 42 and the position of the end of the hydrophobic nonwoven cloth 43 almost match, and at the free end of the barrier cuffs G (that is, the end of the outer side in the width direction W), the hydrophobic nonwoven cloth 43 is folded back such that it sandwiches the elastic members 41 for the barrier cuffs. Furthermore, the water-resistant film 42 does not reach up to the free end of the barrier cuffs G.

A fabric made from a hydrophobic nonwoven cloth made of fibers such as polyolefin and polyethylene terephthalate, which is manufactured by methods such as spun bonding can be used as the hydrophobic nonwoven cloth 43. For example, the hydrophobic nonwoven cloth 43 is 15 g/m² of hydrophobic SMS nonwoven cloth.

Furthermore, a film made from polyethylene, polyethylene terephthalate, and the like can be used as the water-resistant film 42, for example, 18 g/m² of moisture-permeable polyethylene film can be used.

The elastic members 41 for the barrier cuffs may be configured by natural rubber, synthetic rubber, and spandex, etc., for example, the elastic members 41 for the barrier cuffs comprise two 620-tex spandex threads each at the left and right sides, which are fixed by a hot-melt adhesive coated on the hydrophobic nonwoven cloth 43 with the slit nozzle method when extended with an extension rate of 2.2 times.

Furthermore, as shown in Fig. 2 to Fig. 5, the chassis 3 is configured by a center sheet 50, a front waistline sheet 60, and a rear waistline sheet 70.

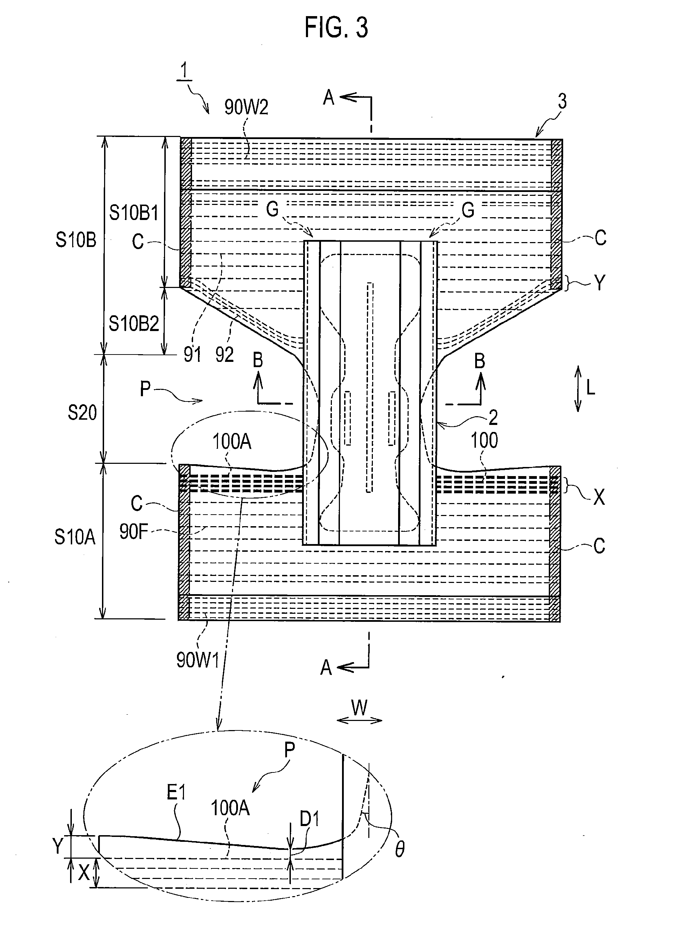

Therefore, as shown in Fig. 3, the chassis 3 comprises a front waistline portion S10A corresponding to the front waistline sheet 60, a rear waistline portion S10B corresponding to the rear waistline sheet 70, and a crotch portion S20 provided between the front waistline portion S10A and rear waistline portion S10B.

Thus, as shown in Fig. 2 to Fig. 5, the absorbent body 2 is provided at the skin contact surface side of the absorbent article 1 of the chassis 3 from the crotch portion S20 across the front waistline portion S10A and rear waistline portion S10B.

Note that as shown in Fig. 3, in the front waistline portion S10A and rear waistline portion S10B, the absorbent body 2 is provided in the central portion in the width direction W.

Furthermore, in the absorbent article 1 according to the embodiment, the configuration is such that respective one of both-side edges C of the front waistline portion S10A and respective one of both-side edges C of the rear waistline portion S10B are joined.

The center sheet 50 is arranged at the skin contact surface side of the front waistline sheet 60 and the rear waistline sheet 70, and is, for example, configured by 15 g/m² of SMS nonwoven cloth made from polypropylene (PP).

Further, the front waistline sheet 60 is configured by a front waistline topsheet 61 and a front waistline backsheet 62.

Here, between the front waistline topsheet 61 and the front waistline backsheet 62, elastic members 90W1 for the hip, elastic members 90F for the front waistline, and elastic members 100 for the leg holes are retained in the extended condition.

Further, as shown in Fig. 4, the front waistline backsheet 62 is folded back towards the inner side in the longitudinal direction L at the end in the longitudinal direction L, and the elastic members 90W1 for the hip are retained in the extended condition in the front waistline backsheet 62 in the folded-back portion.

Similarly, the rear waistline sheet 70 is configured by a rear waistline topsheet 71 and a rear waistline backsheet 72.

Here, between the rear waistline topsheet 71 and the rear waistline backsheet 72, elastic members 90W2 for the hip, elastic members 91 for the rear waistline, and elastic members 92 for the leg holes are retained in the extended condition.

Further, as shown in Fig. 4, the rear waistline backsheet 72 is folded back towards the inner side in the longitudinal direction L at the end in the longitudinal direction L, and the elastic members 90W2 for the hip are retained in the extended condition in the rear waistline backsheet 72 in the folded-back portion.

A sheet made from a hydrophobic nonwoven cloth such as polyolefin and polyethylene terephthalate, which is manufactured by methods such as spun bonding and air-through can be used as the front waistline sheet 60 and the rear waistline sheet 70.

Further, the basis weight of the front waistline topsheet 61, the basis weight of the front waistline backsheet 62, the basis weight of the rear waistline topsheet 71, and the basis weight of the rear waistline backsheet 72 are each desired to be 13 to 30 g/m².

For example, the front waistline topsheet 61 and the rear waistline topsheet 71 are 15 g/m² of SMS nonwoven cloths made from polypropylene, and the front waistline backsheet 62 and the rear waistline backsheet 72 are 17 g/m² of spun bond nonwoven cloths made from polypropylene.

Furthermore, natural rubber, synthetic rubber, or spandex can be used for the elastic members 90W1 and 90W2 for the hip, the elastic members 90F for the front waistline, and the elastic members 91 for the rear waistline, and the extension rate thereof is desired to be 1.3 to 4.0 times (in the case of spandex, the thickness is desired to be in the range of 300 to 1300 dtex).

For example, the extension rate of the elastic members 90W1 and 90W2 for the hip is 3.0 times (the thickness, when spandex is used, is 940 dtex), and the extension rate of the elastic members 90F for the front waistline, and the elastic members 91 for the rear waistline is 2.5 times (the thickness, when spandex is used, is 780 dtex).

For example, the number of elastic members 90W1 and 90W2 for the hip is five each, the number of elastic members 90F for the front waistline is eight, and the number of elastic members 91 for the rear waistline is 13.

Further, the elastic members 100 for the leg hole are arranged in an almost straight line between the two ends in the width direction W in the front waistline topsheet 61 and the front waistline backsheet 62.

In other words, the plurality of elastic members 100 for the leg holes are arranged to extend in an almost straight line in the width direction W of the absorbent article 1, in a first portion X within the front waistline topsheet 61 (that is, the front waistline portion S10A).

For example, the extension rate of the elastic members 100 for the leg holes is 2.0 times (the thickness, when spandex is used, is 780 dtex), and the number of the elastic members 100 for the leg holes is four.

As shown in Fig. 3, in the front waistline portion S10A, the distance D1 between the elastic member (that is, an elastic member for the leg holes) 100A that is closest to the leg hole opening side from among the plurality of elastic members (for example, the elastic members 90W1 for the hip, the elastic members 90F for the front waistline, and the elastic members 100 for the leg hole) extending in an almost straight line in the width direction W, and the edge E1 at the leg hole opening P side of the front waistline portion S10A is configured to increase gradually after decreasing gradually from the both-side edges C towards the center in the width direction W.

For example, the minimum value of the distance D1 is approximately 5 mm. Furthermore, for example, the angle formed between the line along the cut shape of the leg hole opening P in the crotch portion S20 and the longitudinal direction L is less than 30°, and is desired to be in the range of 5° to 25°.

Furthermore, as shown in Fig. 3, the rear waistline portion S10B comprises a hip portion S10B1 and a leg hole portion S10B2.

The hip portion S10B1 is a portion in which the both-side edges C are joined with the both-side edges C of the front waistline portion S10A and which extends from the edge C at one side to the edge C at the other side in the width direction W. Furthermore, the leg hole portion S10B2 is the portion closer to the crotch portion S20 than the hip portion S10B1.

A plurality of elastic members (that is, elastic members for the leg holes) 92 extend from the leg hole portion S10B2 up to the hip portion S10B1, along the leg hole opening P.

That is, the elastic members 92 for the leg holes are arranged in the rear waistline topsheet 71 and the rear waistline backsheet 72, from the end at one side in the width direction W towards the end at the other side while curving along the inner ends in the longitudinal direction L (the portion forming the leg hole opening P).

For example, the extension rate of the elastic members 92 for the leg holes is 1.8 times (the thickness, when spandex is used, is 780 dtex).

Furthermore, the first portion X in which the plurality of elastic members (that is, the elastic members for the leg holes) 100 including the elastic member (that is, an elastic member for the leg holes) 100A that is closest to the leg hole opening P are arranged in the front waistline portion S10A is configured to be joined with a second portion Y in which the plurality of elastic members (that is, the elastic members for the leg hole) 92 are arranged in the hip portion S10B1, in the both-side edges C.

According to the configuration, when the absorbent article 1 is worn, the connectivity between the elastic members 100 for the leg holes in the front waistline portion S10A and the elastic members 92 for the leg holes in the rear waistline portion S10B can be improved, and at the same time, the fitting of the leg hole opening P can be improved, which makes it difficult for the absorbent article to shift from its place.

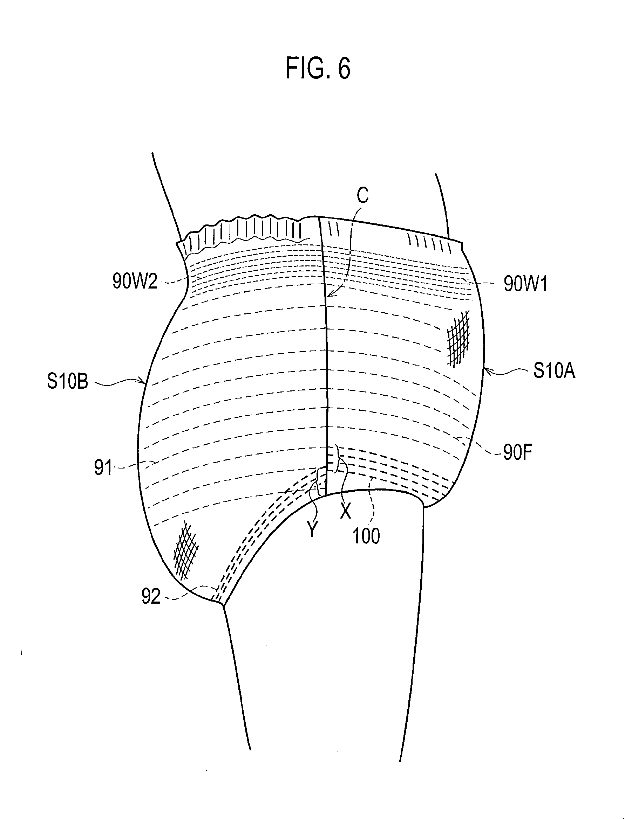

As shown in Fig. 6, the second portion Y is configured to be joined with the first portion X in a state in which the second portion is shifted towards the leg hole opening P side from the first portion X, in the both-side edges C.

According to the configuration, when the absorbent article 1 is worn, the fitting near the lower end of the both-side edges C of the rear waistline portion S10B can be improved, and at the same time, the flipping up in neighboring part of the inguinal portion of the wearer can be reduced.

According to the configuration, in the joined portion where force could be exerted easily, which was a problem in the conventional absorbent article, particularly, in the lower end of the joined portion at the leg hole opening P side in which overlapping of the material is lesser as compared to the longitudinal direction end sides of the front waistline portion S10A and the rear waistline portion S10B, because a portion in which the elastic members are not provided is arranged in the front waistline portion S10A, the sealability improves, resulting in an effect that the joining strength is easy to achieve.

Therefore, even when the basis weight of the sheet material configuring the waistline portion is reduced, a strength that can be withstood at the time of wearing the absorbent article is secured easily.

The elastic members (that is, the elastic members for the leg holes) 100 arranged in the first portion X are arranged so as not to overlap the elastic members (that is, the elastic members for the leg holes) 92 arranged in the second portion Y, in the side edges C.

According to the configuration, due to the overlapping of the elastic members 100 for the leg holes with the elastic members 92 for the leg holes, the occurrence of breakages during joining can be reduced due to the thickening of the joined portion of the both-side edges C of the front waistline portion S10A and the both-side edges C of the rear waistline portion S10B, and thus, a more stable joining strength can be achieved.

Furthermore, the center sheet 50 is configured from a material that is stretched easily in the longitudinal direction L as compared to the material of the front waistline sheet 60 and the rear waistline sheet 70.

Note that as shown in Fig. 2 to Fig. 5, each of the members configuring the above-mentioned center sheet 50, the front waistline sheet 60, and the rear waistline sheet 70 are configured to be joined by a heat seal, a sonic seal, a hot-melt adhesive, and the like.

Furthermore, the front waistline topsheet 61 and the front waistline backsheet 62 are joined by a hot-melt adhesive that is coated directly onto the elastic members 90F for the front waistline and the elastic members 100 for the leg holes, and the rear waistline topsheet 71 and the rear waistline backsheet 72 are joined by a hot-melt adhesive that is coated directly onto the elastic members 91 for the rear waistline.

That is, in the absorbent article 1 according to the embodiment, the hot-melt adhesive is not coated onto the front waistline topsheet 61 and the front waistline backsheet 62. According to the configuration, the softness of the front waistline topsheet 61 and the front waistline backsheet 62 can be improved.

Also, the hot-melt adhesive is not coated onto the rear waistline topsheet 71 and the rear waistline backsheet 72 in the portion where the elastic members 91 for the rear waistline are joined. According to the configuration, the softness of the rear waistline topsheet 71 and the rear waistline backsheet 72 in the portion can be improved.

On the other hand, the hot-melt adhesive may be coated through spiral coating along the pattern of the elastic members 92 for the leg holes onto the rear waistline backsheet 72 in the portion where the elastic members 92 for the leg holes are joined.

In the rear waistline backsheet 72, a hot-melt adhesive may be coated through spiral coating along the width direction W in order to prevent an opening in the edges at the leg hole opening P side along the elastic members 90F for the front waistline.

Note that at locations where the above-mentioned interval of the elastic members is, for example, more than 10 mm, the hot-melt adhesive may be coated partially with coating methods such as spiral coating and control seam.

Rubber adhesives made from styrene-isoprene-styrene (SIS), styrene-butadiene-styrene (SBS), and styrene-ethylene/butylene-styrene (SEBS), as well as olefin adhesives are used as the hot-melt adhesive.

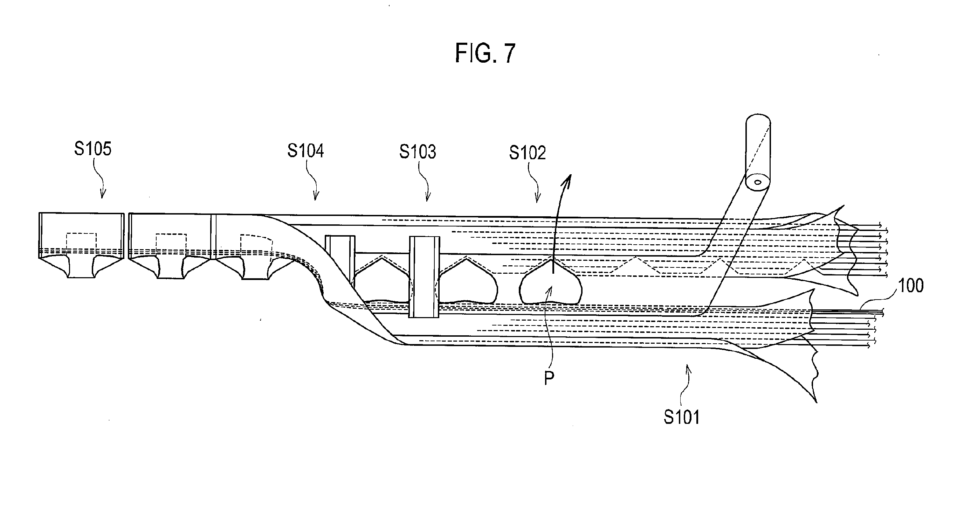

Next, some of the methods of manufacturing the absorbent article 1 according to the embodiment are described with reference to Fig. 7. Note that as far as the methods that are not described in Fig. 7 are concerned, the existing methods can be used.

As shown in Fig. 7, in step S101, the chassis 3 is generated, and in step S102, the leg hole opening P is formed by cutting together the center sheet 50, the front waistline sheet 60, and the rear waistline sheet 70 in the predetermined shape.

In step S102, in the front waistline portion S10A, the center sheet 50, the front waistline sheet 60, and the rear waistline sheet 70 are cut such that the distance D1 between the elastic member 100A for the leg holes and the edge E1 at the leg hole opening P side of the front waistline portion S10A is configured to increase gradually after decreasing gradually from the both-side edges C towards the center in the width direction W.

In step S103, the absorbent body 2 is arranged between the leg hole openings P formed in step S102.

In step S104, after folding the front waistline sheet 60 towards the rear waistline sheet 70, the front waistline sheet 60 and the rear waistline sheet 70 are joined.

In step S105, by cutting the joined portion of the front waistline sheet 60 and the rear waistline sheet 70, the absorbent article 1 is generated.

According to the absorbent article 1 of the present embodiment, by forming the distance D1 between the elastic member 100A for the leg holes and the edge E1 at the leg hole opening P side of the front waistline portion S10A to increase gradually after decreasing gradually from the both-side edges C of the front waistline portion S10A towards the center in the width direction W, it is possible to eliminate the discomfort caused by the difficulty in moving the legs by the wearer while shortening the length of the elastic members 100 for the leg holes in the front waistline portion S10A, and to cover the neighboring part of the inguinal portion of the wearer.

### (First Modification)

Further, the configuration of the chassis 3 and the absorbent body 2 may be different from the configuration of the absorbent article 1 according to the above-mentioned first embodiment.

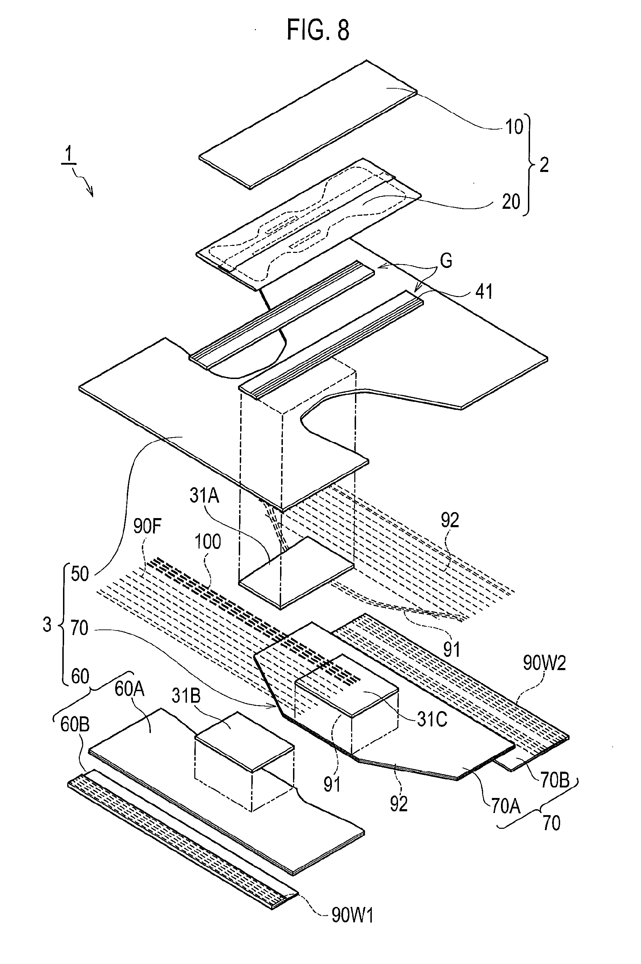

For example, as shown in Fig. 8, instead of the absorbent body side backsheet 30 that is provided inside the absorbent body 2 in the absorbent article 1 according to the first embodiment, leakage-preventing films 31A to 31C may be provided at the chassis 3 side as the leakage-preventing film that must be provided at the skin non-contact surface side of the absorbent article 1 of the absorber core 20.

Note that in the example shown in Fig. 8, the front waistline sheet 60 is configured by a sheet 60A and a sheet 60B, and the rear waistline sheet 70 is configured by a sheet 70A and a sheet 70B.

In the absorbent article 1 having the configuration, the absorbent body 2 is configured by the absorber core 20 and the absorbent body side topsheet 10.

Further, the chassis 3 and the absorbent body 2 may even be integrated. In the absorbent article 1 having the configuration, the absorbent body 2 is configured by the absorber core 20. That is, the absorbent body side topsheet 10 and the absorbent body side backsheet 30 are not included in the absorbent body 1.

Thus, the present invention has been explained in detail by using the above-described embodiments; however, it is obvious that for persons skilled in the art, the present invention is not limited to the embodiments explained herein. The present invention can be implemented as corrected and modified modes without departing from the gist and the scope of the present invention defined by the claims. Therefore, the description of the specification is intended for explaining the example only and does not impose any limited meaning to the present invention.

In addition, the entire content of Japanese Patent Application No. 2010-096532 (filed on April 19, 2010) is incorporated in the present specification by reference.

### INDUSTRIAL APPLICABILITY

As described above, it is possible to provide an absorbent article which may eliminate the discomfort caused by a decline in the feeling of wearing the absorbent article while shortening the length of the elastic members for the leg holes, and to cover the neighboring part of the inguinal portion of the wearer.

### REFERENCE SIGNS LIST

- 1: Absorbent article
- S10A: Front waistline portion
- S10B: Rear waistline portion
- S2C: Crotch portion
- 2: Absorbent body
- 3: Chassis
- 90W1, 90W2: Elastic members for the hip
- 90F: Elastic members for the front waistline
- 91: Elastic members for the rear waistline
- 92, 100: Elastic members for the leg holes

## Claims

1. An absorbent article, comprising: a chassis; and an absorbent body, wherein
the chassis includes a front waistline portion, a rear waistline portion, and a crotch portion provided between the front waistline portion and the rear waistline portion,
respective one of both-side edges of the front waistline portion and respective one of both-side edges of the rear waistline portion are configured to be joined, and
in the front waistline portion, a distance between an elastic member closest to a leg hole opening from among a plurality of elastic members extending in an almost straight line in a width direction and an edge at the leg hole opening side of the front waistline portion is configured to increase gradually after decreasing gradually from the both-side edges towards a center in the width direction.

2. The absorbent article according to claim 1, wherein the rear waistline portion includes: a hip portion in which the both-side edges of the rear waistline portion are joined with the both-side edges of the front waistline portion and which extends from an edge at one side of the rear waistline portion to an edge at the other side of the rear waistline portion in the width direction; and a leg hole portion that is closer to the crotch portion than the hip portion,
the plurality of elastic members extend from the leg hole portion up to the hip portion along the leg hole opening, and
a first portion in which a plurality of elastic members including the elastic member closest to the leg hole opening are arranged in the front waistline portion is configured to be joined with a second portion in which the plurality of elastic members are arranged in the hip portion, in the both-side edges.

3. The absorbent article according to claim 2, wherein the second portion is configured to be joined with the first portion in a state in which the second portion is shifted towards the leg hole opening side from the first portion, in the both-side edges.

4. The absorbent article according to claim 2, wherein the elastic members arranged in the first portion are arranged so as not to overlap the elastic members arranged in the second portion, in the side edges.
